# EUROPEAN PATENT APPLICATION

(11) **EP 4 685 241 A1**
(43) Date of publication of application: **28.01.2026**
(21) Application number: 23928896.2
(22) Date of filing: 27.12.2023
(51) Int. Cl.: C12Q 1/6883, G01N 33/68

(54) **BIOMARKER COMPOSITION FOR DIAGNOSIS OF OPTIC NERVE INJURY DISEASES AND USE THEREOF**

(30) Priority: 21.03.2023 KR 20230036709
(71) Applicant: Sungkwang Medical Foundation, Seoul 06135 (KR); Cha University Industry-Academic Cooperation Foundation, Pocheon-si, Gyeonggi-do 11160 (KR)
(72) Inventor: LEW, Helen, Seoul 05510 (KR); PARK, Mira, Seoul 01672 (KR); SHIN, Hyun-Ah, Seongnam-si, Gyeonggi-do 13500 (KR)
(74) Representative: Weickmann & Weickmann PartmbB
(86) International application number: PCT/KR2023/021671
(87) International publication number: WO 2024/195973

(57) **Abstract**

According to a composition, kit, and method for measuring an expression level of STX 12 and Delphilin protein, a fragment thereof, or a polynucleotide encoding the protein, according to an aspect, optic nerve injury-related diseases may be efficiently diagnosed to maximize therapeutic effects using an optic nerve injury therapeutic agent, and candidate substances capable of treating optic nerve injury may be efficiently screened.

## Description

### Technical Field

The present disclosure relates to a composition for diagnosing optic nerve injury diseases, including a preparation for measuring the expression level of a protein of Syntaxin 12 or Delphilin, a fragment thereof, or a polynucleotide encoding the protein. The present application claims priority to Korean Patent Application No. 10-2023-0036709, filed on March 21, 2023, with the Korean Intellectual Property Office, the disclosure of which is incorporated in the present specification by reference.

### Background Art

The human optic nerve is made up of about 1 million optic nerve fibers, and it is known that when 30 % of them are injured, abnormalities may be detected in the static field of vision, and when 50 % of them are injured, the first effects on the visual field are detected in the dynamic field of vision. Currently, stem cell therapy and various other treatments are being used to treat optic nerve injury, but there are no clear recovery indicators to verify the recovery function of these treatments.

Biomarkers are generally indicators that may detect changes in the body using proteins, DNA, RNA, metabolites, etc. In other words, they refer to markers that may distinguish between normal and pathological states in the case of specific diseases and conditions, or may predict treatment responses and be objectively measured.

Against this technical background, the researchers of the present disclosure attempted to discover a biomarker as a recovery indicator to diagnose optic nerve diseases and verify the recovery function of optic nerve injury treatment.

### Disclosure of Invention

### Technical Problem

One aspect is to provide a composition for diagnosing an optic nerve injury disease, including a preparation for measuring the expression level of a protein of Syntaxin 12 or Delphilin, a fragment thereof or a polynucleotide encoding the protein.

Another aspect is to provide a kit for diagnosing optic nerve injury disease including the above composition.

Another aspect is to provide an information provision method for diagnosing or treating an optic nerve injury disease, including: measuring the expression level of STX 12 or Delphilin protein, a fragment thereof, or a polynucleotide encoding the protein in a biological sample isolated from an individual; and
comparing the measured expression level of the protein, fragment thereof, or polynucleotide encoding the protein with that of a normal control sample.

Another aspect is to provide a method of screening for a therapeutic agent for optic nerve injury, including: measuring the expression level of STX 12 or Delphilin protein, a fragment thereof, or a polynucleotide encoding the protein in a biological sample isolated from an individual administered with a candidate substance for treating optic nerve injury; and
comparing the measured expression level of the protein, fragment thereof, or polynucleotide encoding the protein with that of a control sample not administered with the candidate substance.

### Solution to Problem

One aspect is to provide a composition for diagnosing an optic nerve injury disease, including a preparation for measuring the expression level of a protein of Syntaxin 12 (STX 12) or Delphilin, a fragment thereof, or a polynucleotide encoding the protein.

As used herein, the term "Syntaxin (STX)" refers to a member of the soluble N-ethylmaleimide sensitive factor attachment protein receptor (SNARE) protein family, which primarily regulates membrane fusion in eukaryotic cells. When Syntaxin is expressed in different tissues, it respectively exhibits different functions and exists in various isoforms. For example, Syntaxin 1A plays an important role in synaptic vesicle fusion in neurons, and Syntaxin 4 is involved in glucose uptake in fat cells and muscle cells. In the present application, Syntaxin may refer to a Syntaxin protein mainly expressed in retinal tissue.

As used herein, the term "Delphilin" refers to a member of the Homer family of proteins that regulate synaptic function, primarily in the brain. "Delphilin" is mainly present in the postsynaptic density (PSD) of excitatory synapses, and is known to interact with glutamate receptor subunits of the GluN2A and GluN2B groups and other PSD protein families such as PSD-95. In the present application, Delphilin may be expressed mainly in retinal tissue.

As used herein, the term "optic nerve injury disease" may be caused by trauma, post-surgery, ischemia, hypoxia, metabolism, infection, drug addiction, immunity, inflammation, genetic factors, tumors or brain lesions, etc., and may be, for example, traumatic optic neuropathy, ischemic optic neuropathy, compressive optic neuropathy, toxic optic neuropathy, Leber hereditary optic neuropathy, optic nerve transection, optic neuritis, neuromyelitis optica or amblyopia, but is not limited thereto. According to an embodiment, the optic nerve injury disease may be caused by hypoxia, and more specifically, may be ischemic optic neuropathy or compressive optic neuropathy.

The term "hypoxia" may refer to a condition of low oxygen tension compared to the common normoxic condition of 21 % oxygen tension. In an example, a hypoxic injury environment was created with CoCl₂.

As used herein, the term "measuring the expression level" may be determined by determining the expression level of a polynucleotide of STX 12 or Delphilin, or a protein encoded by the polynucleotide, or a fragment thereof.

As used herein, the term "preparation" may include a primer, probe, nucleotide, antibody or antigen-binding fragment thereof, ligand, receptor, protein or a combination thereof that specifically binds to STX12 or Delphilin protein, a fragment thereof or a polynucleotide encoding said protein.

As used herein, the term "primer" refers to a nucleic acid sequence having a free 3' hydroxyl group that may form base pairs with a template complementary to a specific nucleotide sequence and serves as a starting point for copying the template strand. The primer may initiate DNA synthesis in the presence of a polymerization reagent (in other words, DNA polymerase or reverse transcriptase) and four different nucleoside triphosphates in an appropriate buffer and temperature. For example, optic nerve injury may be diagnosed by measuring the amount of desired product produced by performing PCR amplification using sense and antisense primers having a sequence of 7 to 50 nucleotides as specific primers for the mRNA of STX 12 or Delphilin polynucleotide. PCR conditions, sense and antisense primer lengths may be appropriately selected according to techniques known in the art.

As used herein, the term "probe" refers to a nucleic acid fragment such as RNA or DNA that may specifically bind to a target nucleic acid, for example, mRNA, and may be labeled so as to confirm the presence or absence, content, and expression level of a specific mRNA. Probes may be produced in the form of oligonucleotide probes, single strand DNA probes, double strand DNA probes, RNA probes, etc. For example, by performing hybridization using a probe having a nucleic acid sequence complementary to the mRNA of STX 12 or Delphilin, the level of mRNA expression may be measured through the degree of hybridization, thereby diagnosing injury to the optic nerve of an individual. Selection of an appropriate probe and hybridization conditions may be appropriately selected according to techniques known in the art.

Additionally, primers or probes may be chemically synthesized using phosphoramidite solid support synthesis or other well-known methods. Additionally, these nucleic acid sequences may be modified through various methods known in the art. Examples of such modifications may include methylation, capping, substitution with one or more analogs of natural nucleotides, or modifications between nucleotides, for example, uncharged linkages (for example, methyl phosphonate, phosphotriester, phosphoramidate, carbamate, etc.) or charged linkages (for example, phosphorothioate, phosphorodithioate, etc.). Additionally, primers or probes may be modified with labels that may directly or indirectly provide a detectable signal. Examples of such labels may include radioactive isotopes, fluorescent molecules, or biotin.

As used herein, the term "antibody" is a term known in the art and refers to a specific immunoglobulin directed against an antigenic site. The above antibody may specifically bind to STX 12 or Delphilin protein or a fragment thereof. The protein fragment of STX 12 or Delphilin may be, for example, an immunogenic fragment. Here, fragment refers to a protein fragment having one or more epitopes that may be recognized by antibodies to STX 12 or Delphilin protein. A polynucleotide of STX 12 or Delphilin may be cloned into an expression vector, and an STX 12 or Delphilin protein encoded by the polynucleotide may be acquired, and an antibody may be produced from the acquired protein according to a typical method in the art.

The form of the above antibody includes a polyclonal antibody, a monoclonal antibody, or a recombinant antibody, and includes all immunoglobulin antibodies. The above antibody refers to a complete form having two full-length light chains and two full-length heavy chains. Additionally, the above antibodies include special antibodies such as humanized antibodies. Polyclonal antibodies may be prepared by injecting the immunogenic biomarker protein or fragment thereof into a foreign host according to conventional methods known to a person of ordinary skill in the art. External hosts may be mammals such as mice, rats, sheep, and rabbits. When the above immunogen is injected intramuscularly, intraperitoneally or subcutaneously, it may generally be administered together with an adjuvant to increase antigenicity. Subsequently, blood may be drawn periodically from the external host to collect serum showing enhanced titer and specificity for the antigen, or antibodies may be isolated and purified therefrom.

Monoclonal antibodies may be produced by techniques for preparing immortalized cell lines by fusion known to a person of ordinary skill in the art. Briefly describing the method of preparing a monoclonal antibody, the protein is purified and an appropriate amount of about 10 µg is immunized into a Balb/C mouse, or a polypeptide fragment of the protein is synthesized and combined with bovine serum albumin to immunize a mouse, and then antigen-preparing lymphocytes isolated from the mouse are fused with human or mouse myeloma to produce an immortalized hybridoma, and only hybridoma cells preparing the desired monoclonal antibody are selected and proliferated using an enzyme linked immunosorbent assay (ELISA) method, and the monoclonal antibody may be isolated and purified from the culture. Additionally, monoclonal antibodies may be obtained and used commercially as antibodies against STX12 or Delphilin proteins.

Using these antibodies, it is possible to determine whether the protein is expressed in a biological sample by methods known in the art, such as enzyme linked immunosorbent assay (ELISA), radioimmunoassay (RIA), sandwich assay, Western blotting on polyacrylic gel, or immunoblotting, etc.

According to an embodiment, it was confirmed that the expression of STX 12 and Delphilin proteins among various proteins was significantly reduced in retinal progenitor cells in which optic nerve injury was induced by hypoxia, and it was confirmed that the proteins are related to optic nerve injury disease. In addition, it was confirmed that the group administered with the optic nerve injury treatment showed a significant recovery in the protein and mRNA expression of STX 12 and Delphilin compared to the group that was not administered with the treatment. Therefore, it was confirmed that the STX 12 or Delphili protein according to the present disclosure and the polynucleotide encoding the protein may serve as an indicator biomarker for optic nerve injury and recovery.

Another aspect is to provide a kit for diagnosing optic nerve injury disease including the above composition.

Any term or element mentioned in the above kit that is the same as that mentioned in the description of the above composition is understood to be the same as that mentioned in the description of the above composition.

In an embodiment, the kit may be a reverse transcription polymerase chain reaction (RT-PCR) kit, a DNA chip kit, an enzyme linked immunosorbent assay (ELISA) kit, a protein chip kit, a rapid kit, or a multiple reaction monitoring (MRM) kit, and the kit may be a kit including essential elements necessary for measuring the expression level of a protein or mRNA.

According to an embodiment, the kit is effective in rapidly diagnosing optic nerve injury by measuring the expression level of STX 12 or Delphilin protein, or a polynucleotide thereof.

Another aspect is to provide an information provision method for diagnosing or treating an optic nerve injury disease, including: measuring the expression level of STX 12 or Delphilin protein, a fragment thereof, or a polynucleotide encoding the protein in a biological sample isolated from an individual; and
comparing the measured expression level of the protein, fragment thereof, or polynucleotide encoding the protein with that of a normal control sample.

Any term or element mentioned in the above information provision method that is the same as that mentioned in the description of the composition or kit is understood to be the same as that mentioned in the description of the composition above.

As used herein, the term "subject" refers to an individual for whom it is desired to confirm or diagnose whether or not an optic nerve injury disease exists. The above subject may be a vertebrate, specifically a mammal, amphibian, reptile, bird, etc., and more specifically a mammal, for example, a human (Homo sapiens).

As used herein, the term "sample" may include whole blood, serum, plasma, saliva, urine, sputum, lymph, cells, tissues, etc. separated from an individual, and in the present application, in the case of tissue, it may be retinal tissue, and in the case of cells, it may be optic nerve cells.

The method may include determining that the subject has an optic nerve injury disease if the expression level is decreased compared to the expression level of a normal control group.

According to an embodiment, if the expression level of STX 12 or Delphilin protein, a fragment thereof, or a polynucleotide encoding the protein measured from retinal tissue is decreased compared to a normal control, the subject may be determined to have an optic nerve injury disease.

Another aspect is to provide a method of screening for a therapeutic agent for optic nerve injury, including: measuring the expression level of STX 12 or Delphilin protein, a fragment thereof, or a polynucleotide encoding the protein in a biological sample isolated from an individual administered with a candidate substance for treating optic nerve injury; and
comparing the measured expression level of the protein, fragment thereof, or polynucleotide encoding the protein with that of a control sample not administered with the candidate substance.

Any term or element mentioned in the above screening method that is the same as that mentioned in the description of the composition, kit or method is understood to be the same as that mentioned in the description of the composition, kit or method above.

As used herein, the term "candidate substance" may include, without limitation, a newly synthesized or known substance that is expected to be effective in treating optic nerve injury.

"Administration" of the above candidate drug refers to introducing a certain substance into an individual by an appropriate method. The above candidate substance may be administered via any common route as long as it may reach the target tissue. It may be administered intraperitoneally, intravenously, intramuscularly, subcutaneously, intradermally, orally, topically, intranasally, intrapulmonary, or rectally.

In the above method, if the expression level of the protein of STX 12 or Delphilin, a fragment thereof, or a polynucleotide encoding the protein measured from a sample of an individual is increased compared to a control group with optic nerve injury, the candidate substance may be determined as a treatment for optic nerve injury disease. In an example, after administration of placental-derived mesenchymal stem cells or human-derived optic nerve progenitor cells, the protein expression level of STX 12 or Delphilin was increased compared to the control group with injured optic nerves.

In an embodiment, the measurement of the expression level of the polynucleotide may be by reverse transcription polymerase reaction, competitive reverse transcription polymerase reaction, real-time reverse transcription polymerase reaction, RNase protection assay, Northern blotting, or DNA chip.

In an embodiment, the measurement of the expression level of the protein or fragment thereof may be performed by protein chip analysis, immunoassay, ligand binding assay, matrix desorption/ionization time of flight mass spectrometry (MALDI-TOF) analysis, surface enhanced laser desorption/ionization time of flight mass spectrometry (SELDI-TOF) analysis, radioimmunoassay, radioimmunodiffusion, ouchterlony immunodiffusion, rocket immunoelectrophoresis, tissue immunostaining, complement fixation assay, two-dimensional electrophoresis analysis, liquid chromatography-mass spectrometry (LC-MS), liquid chromatography-mass spectrometry/mass spectrometry (LC-MS), western blot, enzyme linked immunosorbent assay (ELISA), or a combination thereof.

### Advantageous Effects of Invention

According to a composition, kit, and method according to an aspect, optic nerve injury related diseases may be efficiently diagnosed, thereby maximizing the therapeutic effect of a treatment using an optic nerve injury therapeutic agent, and may efficiently screen candidate substances capable of treating optic nerve injury.

### Brief Description of Drawings

FIG. 1A is a graph illustrating the results of proteomics analysis after co-culturing placental-derived mesenchymal stem cells with R28 cells in which optic nerve injury was induced in a hypoxic environment.
FIG. 1B is a graph illustrating the results of proteomics analysis after co-culturing optic nerve progenitor cells with R28 cells in which optic nerve injury was induced in a hypoxic environment.
FIG. 2A is a graph illustrating that the expression levels of STX 12 and Delphilin mRNA significantly increased after treating R28 cells with placental-derived mesenchymal stem cells. *p<0.05, **p<0.01, ***p<0.001 vs CON; #p<0.05, ##p<0.01 vs CoCl₂
FIG. 2B is a graph illustrating that the expression levels of STX 12 and Delphilin mRNA significantly increased after treatment of optic nerve progenitor cells in R28 cells. *p<0.05, **p<0.01, ***p<0.001 vs CON; #p<0.05, ##p<0.01 vs CoCl₂
FIG. 3A is a diagram illustrating the expression level of STX 12 in the optic nerve region on the eye side of an animal model with optic nerve injury, using immunofluorescence staining. The area marked by the arrow is injured by hypoxia.
FIG. 3B is a diagram illustrating the expression level of Tuj1 in the optic nerve region on the eye side of an animal model with optic nerve injury, using immunofluorescence staining. The area marked by the arrow is injured by hypoxia.
FIG. 3C is a diagram illustrating the expression level of Gap43 in the optic nerve region on the eye side of an animal model with optic nerve injury, using immunofluorescence staining. The area marked by the arrow is injured by hypoxia.
FIG. 4A is a diagram illustrating the protein expression levels of STX 12 and Delphilin expressed in the oculus sinistra (OS) and oculus dexter (OD) of an animal model with optic nerve injury, as determined by Western blot analysis.
FIG. 4B is a graph comparing the protein expression levels of STX 12 and Delphilin expressed in the oculus sinistra (OS) of an optic nerve-injured animal model with those of an NPC-treated group, normalized to the protein expression levels in an uninjured oculus dexter (OD). *p<0.05, **p<0.01 vs Sham
FIG. 5 is a graph illustrating the protein expression pattern of Delphilin after 1 week, 2 weeks, or 4 weeks in ischemic or compressive optic neuropathy. *p<0.05, **p<0.01 vs Sham
FIG. 6 is a graph illustrating the protein expression pattern of STX12 after 1 week, 2 weeks, or 4 weeks in ischemic or compressive optic neuropathy. **p<0.01 vs Sham
FIG. 7 is a diagram illustrating the results of immunofluorescence staining of optic nerve tissue of an animal model.
FIG. 8 is a graph illustrating the results of immunofluorescence staining of optic nerve tissue of an animal model. *p<0.05 vs Age-matched sham; #p<0.05, ##p<0.01 vs 1w Sham

### Mode for the Invention

Hereinafter, the present disclosure will be described in more detail through examples. However, these examples are intended to illustrate the present disclosure by way of example and the scope of the present disclosure is not limited to these examples.

### Experimental Example 1. Preparation of hypoxia injured R28 cells

Experiments were conducted using a R28 cell line, a rat retinal progenitor cell line. First, R28 cells are seeded in a 6-well plate at a cell number of 2x10⁵. When the cell density reached approximately 50 %, CoCl₂ was treated at a concentration of 300 µM to create a hypoxic injury environment. After treatment for 3 hours, R28 cells were harvested by co-culture with placental-derived mesenchymal stem cells or human pluripotent stem cell-derived neural progenitor cells (NPCs) for 24 hours.

Hereinafter, R28 cells with hypoxic injury were designated as the experimental group, R28 cells co-cultured with stem cells as the Comparison Group 1, and R28 cells co-cultured with optic nerve progenitor cells as the Comparison Group 2, and a recovery function analysis experiment was conducted.

### Example 1. Identification of proteins associated with optic nerve injury

In the present example, hypoxia injured R28 cells prepared as described above were used to identify proteins associated with optic nerve injury through proteomics analysis.

Proteomics analysis was conducted using experimental group, Comparison Group 1, and Comparison Group 2, and the process was as follows.

Cell lysates prepared in PBS buffer were subjected to protein extraction using a Covaris S2 Focused-Ultrasonicator (Covaris, Woburn, MA, USA). Protease inhibitor was added at 25X, proteins were extracted at 20 °C for 12 to 15 minutes, and the concentration of the solution was measured using BCA analysis. After preparing the total protein weight to be 100 µg, TCEP was added to the sample solution to a final concentration of 5 mM according to the sample volume, and the mixture was incubated at 37 °C for 30 minutes with mixing at 300 rpm. The sample solution was transferred to a YM-30 filter, centrifuge at 14,000 x g, 20 °C for 15 minutes, followed by the addition of 200 µL of UA, centrifuge at 14,000 x g, 20 °C for 20 minutes, and the same process was repeated once. 50 mM iodoacetamide (IAA) in 50 mM ammonium bicarbonate (ABC) was added to the sample solution, mixed at 300 rpm for 30 minutes at 25 °C, light was blocked, and incubated. Centrifuged at 14,000 x g, 20 °C for 15 minutes. 100 µL of UA was added, and centrifuged at 14,000 × g and 20 °C for 15 minutes, and the same process was repeated twice. 100 µL of ABC was added, and centrifuged at 14,000 × g and 20°C for 15 minutes, and the same process was repeated twice. 200 µL of trypsin-including 50 mM ABC was added at an enzyme-to-protein ratio of 1:50, mixed at 600 rpm for 1 minute at 37 °C, and then incubated at 37 °C for 18 hours. After replacing the collection tube with a new one, centrifuged at 14,000 × g and 20 °C for 10 minutes, 40 µL of 50 mM ABC was added, centrifuged at 14,000 × g and 20 °C for 10 minutes, and the same process was repeated once. To adjust the pH to 2-3, 15 µL of 100 % formic acid was added. A reverse phase micro C18 spin column was conditioned by adding 100 µL of 100 % methanol (MeOH) and centrifuging at 3,400 × g and 4 °C for 2 minutes. Subsequently, 100 µL each of 0.1 % formic acid, 80 % ACN in 0.1 % formic acid, and 0.1 % formic acid were sequentially added and centrifuged at 3,400 × g and 4 °C for 2 minutes per solution to equilibrate the column. The sample solution was transferred to the column and loaded by centrifugation at 3,400 × g and 4 °C for 2 minutes. 50 µL of 0.1 % formic acid was added and centrifuged at 3,400 × g and 4 °C for 2 minutes. The column was transferred to a new 1.5 mL microtube, and 100 µL of 0.1 % formic acid including 80 % ACN was added and centrifuged at 3,400 × g and 4 °C for 2 minutes. The sample solution was then dried using a SpeedVac and either stored at -20 °C or proceed with LC-MS analysis.

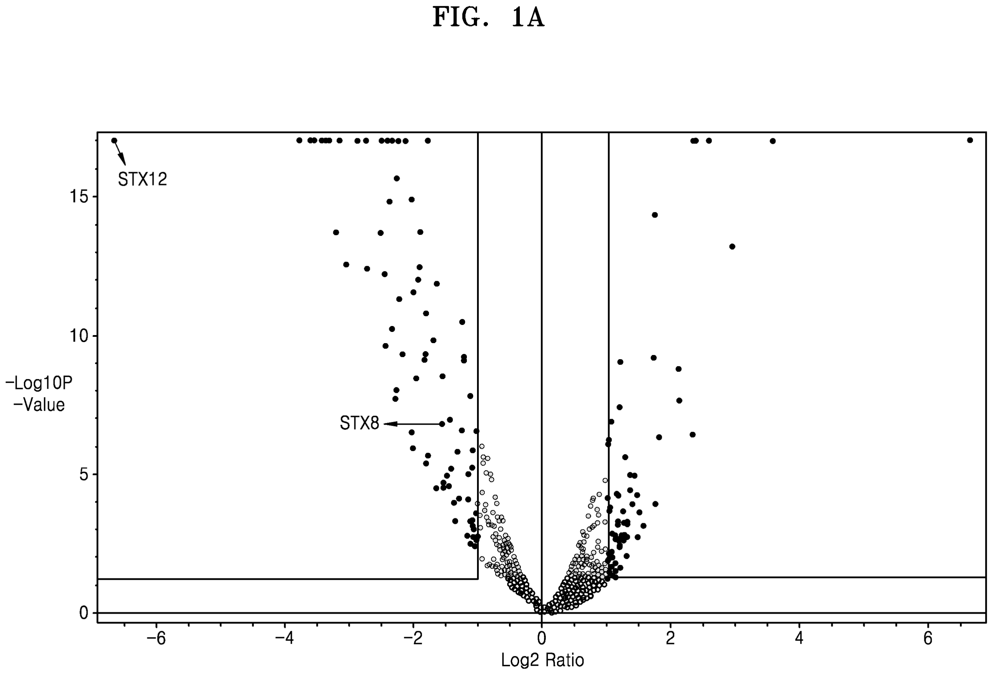

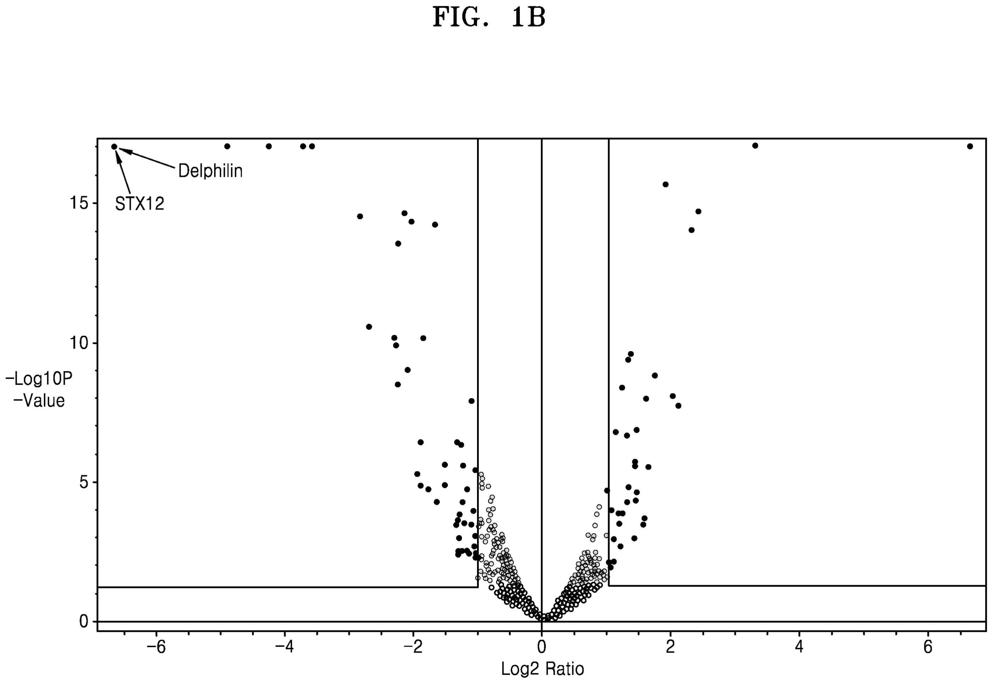

As a result, it was confirmed that the expression of Syntaxin 12 (STX12) in the experimental group that suffered hypoxic injury was significantly lower compared to the Comparison Group 1 that was treated with stem cells (FIG. 1A). In addition, it was confirmed that the expression of STX 12 and Delphilin proteins in the experimental group was significantly lower compared to the Comparison Group 2 treated with progenitor cells (FIG. 1B).

Based on the above results, STX 12 and Delphilin were identified as candidate target proteins differentially regulated by optic nerve injury.

### Example 2. In vitro analysis of polynucleotide expression levels of proteins

In the present example, a recovery function analysis was performed by confirming changes in the polynucleotide expression levels of the optic nerve marker proteins derived in Example 1.

Specifically, R28 cells from the experimental group, Comparison Group 1, or Comparison Group 2 were collected, RNA was isolated, and qRT-PCR was performed using primers for genes related to signaling mechanisms involved in optic nerve recovery, including Syntaxin12 and Delphilin. In the present example, normal cells, R28 cells that were not injured by hypoxia, were used as a control group.

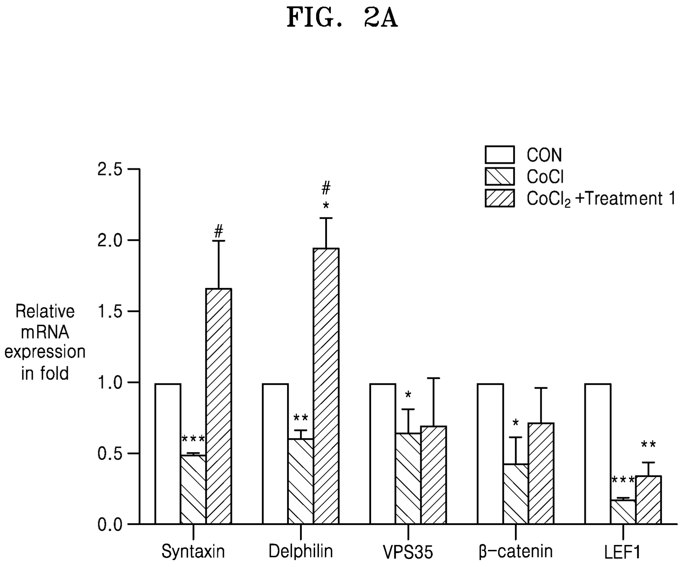

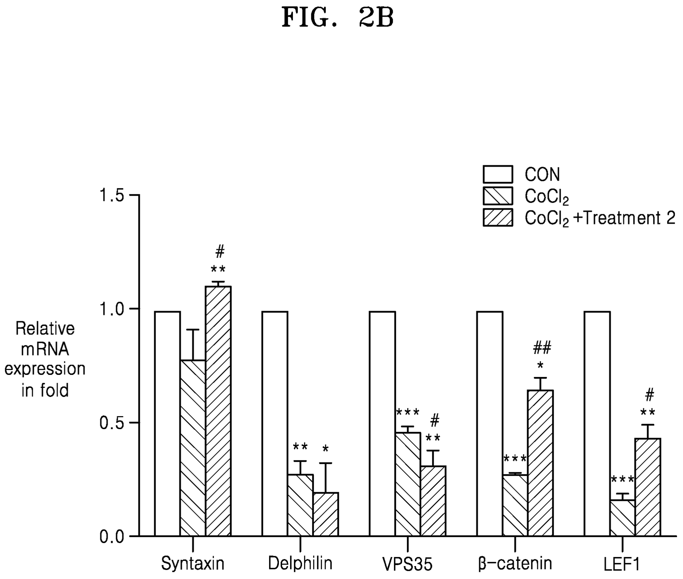

As a result, it was confirmed that the mRNA expression levels of STX 12, Delphilin, and related optic nerve recovery signaling factors (VPS35, β-catenin, LEF1) were all significantly reduced when hypoxic injury was caused, and it was confirmed that the mRNA expression levels of STX 12 and Delphilin were significantly recovered in both the stem cell-treated group (Comparison Group 1) and the optic nerve progenitor cell-treated group (Comparison Group 2) (FIG. 2A and FIG. 2B, respectively).

### Example 3. Analysis of protein expression levels in optic nerve and retina in animal models

In the present example, changes in the optic nerve marker proteins derived in Example 1 were examined in the retina.

An optic nerve injury model was created by compressing the optic nerve tissue of the oculus sinistra (OS) of a 7-week-old rat with forceps for approximately 5 seconds, and optic nerve progenitor cells (NPCs) were injected into the back of the eye of the rat. After 4 weeks, the expression changes of STX 12 and Tuj1 and Gap43 proteins, known as general optic nerve markers, in the optic nerve of the animal model were confirmed using immunostaining and Western blot.

The process for performing immunostaining is as follows.

After reacting the 1^{st} antibody on the optic nerve tissue slide at a dilution ratio of STX 12 (1:50), Tuj1 (1:200), and Gap43 (1:200) overnight at 4 °C, the 2^{nd} antibody Alexa Fluor 488 was reacted at a ratio of 1:200 at room temperature for 90 minutes, followed by DAPI staining and analysis using a fluorescence microscope.

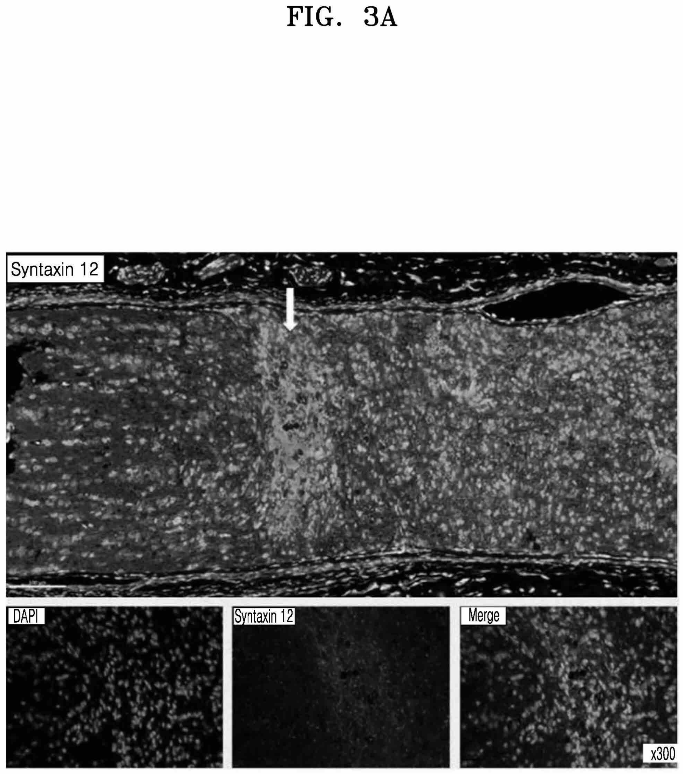

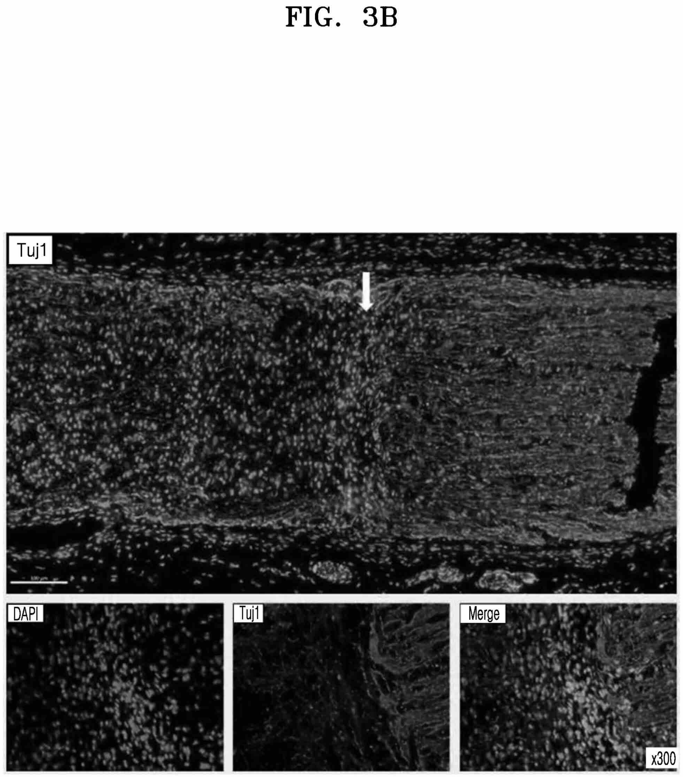

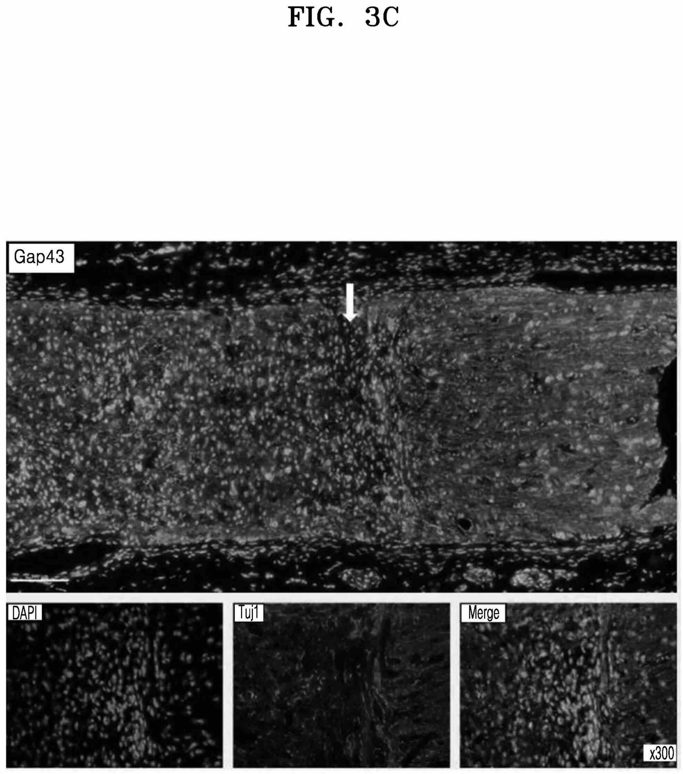

FIG. 3A is a diagram illustrating the expression level of STX 12 in the optic nerve region on the eye side of an animal model with optic nerve injury, FIG. 3B shows the expression level of Tuj1, and FIG. 3C illustrates the expression level of Gap43 using fluorescent immunostaining. The area marked by the arrow is injured by hypoxia.

Immunostaining analysis results confirmed that Syntaxin 12 expression was significantly reduced in the left eye area with injured optic nerve, and this pattern was similar to that of other optic nerve markers, Tuj1 and Gap43 (FIG. 3).

Additionally, in Western blot analysis, the amount of protein expressed in the tissue of the oculus sinistra (OS) with injured optic nerve was normalized to the amount of protein expressed in the uninjured oculus dexter (OD).

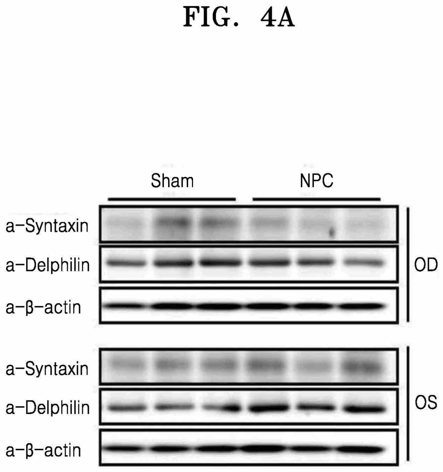

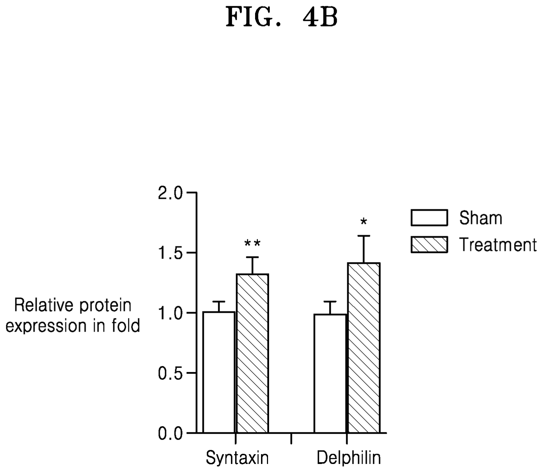

FIG. 4A is a graph illustrating the protein expression levels of STX 12 and Delphilin expressed in the oculus sinistra (OS) and oculus dexter (OD) of an optic nerve-injured animal model confirmed by Western blot, and FIG. 4B is a graph illustrating the protein expression levels of STX 12 and Delphilin expressed in the oculus sinistra (OS) of an optic nerve-injured animal model compared to the NPC-treated group, normalized to the protein levels expressed in the uninjured oculus dexter (OD).

Western blot analysis results confirmed that the expression of STX 12 and Delphilin proteins in the retinal tissue of the NPC-administered group was significantly increased compared to the optic nerve-injured group (Sham: optic nerve compression followed by BSS injection) (FIG. 4).

In summary, the results of Examples 2 and 3 above show that by comparing the expression levels of STX 12 and Delphilin proteins or polynucleotides thereof, optic nerve injury diseases may be efficiently diagnosed, and not only may candidate therapeutic agents for optic nerve injury be efficiently screened, but also the therapeutic effect using the therapeutic agent may be maximized.

### Example 4. Confirmation of diagnostic efficacy of markers in diverse optic nerve injury diseases

In the present example, the diagnostic efficacy of STX 12 and Delphilin in various optic nerve injury diseases was confirmed.

Specifically, animal models of ischemic (ONCo) and compressive (ONCr) optic neuropathy were created by compressing the optic nerve behind the eye of a rat using different tools for approximately 3 seconds. Afterwards, optic nerve progenitor cells (NPCs) were locally injected into the orbit, and the retinal tissue of rats was lysed after 1, 2, or 4 weeks to confirm the difference in expression of the target protein.

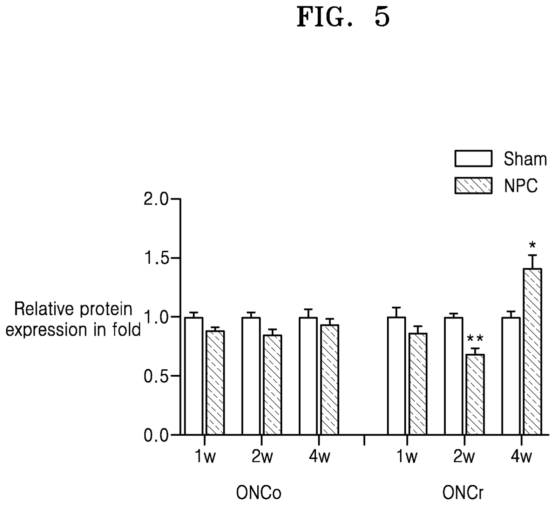

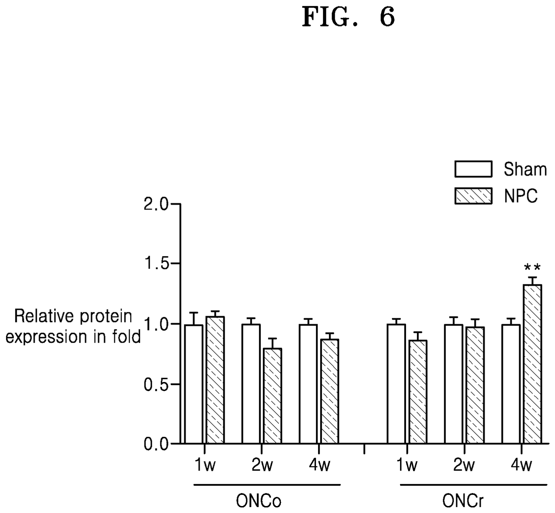

FIG. 5 is a graph illustrating the protein expression pattern of Delphilin after 1 week, 2 weeks, or 4 weeks in ischemic or compressive optic neuropathy, and FIG. 6 is a graph illustrating the protein expression pattern of STX12 after 1 week, 2 weeks, or 4 weeks in ischemic or compressive optic neuropathy.

As a result, it was confirmed that the expression of Delphilin and STX12 was significantly recovered in the 4-week group of the compressive optic neuropathy animal model (FIG. 5 and FIG. 6).

In addition, paraffin blocks were made from the optic nerve tissue of the rat and immunofluorescence staining was performed to confirm the regulation of STX12 protein expression.

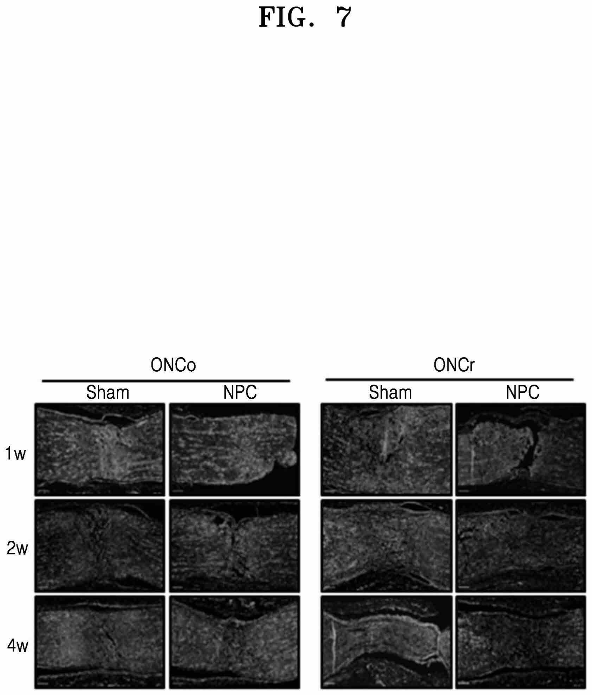

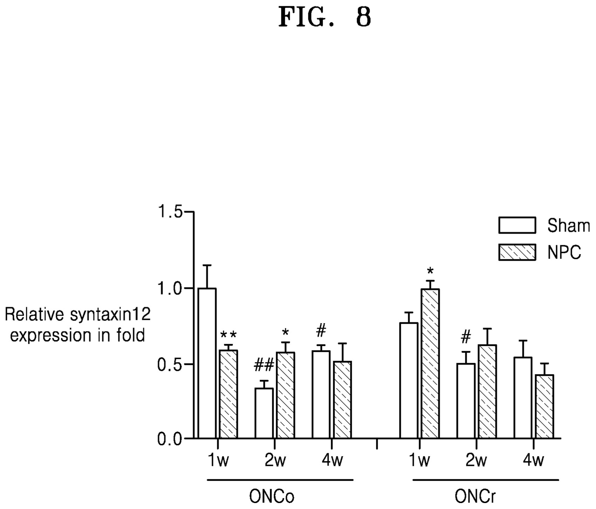

FIG. 7 is a diagram illustrating the results of immunofluorescence staining performed on the optic nerve tissue of the animal model, and FIG. 8 is a graph illustrating those results.

As a result, it was confirmed that expression was significantly restored 1 week after administration of optic nerve progenitor cells in an animal model of compressive optic neuropathy (ONCr) (FIG. 8).

As a result of the above, by comparing the expression levels of STX 12 and Delphilin proteins or their polynucleotides with those of a normal control group, various optic nerve injury diseases such as ischemic and compressive optic neuropathy may be efficiently diagnosed, and not only may candidate therapeutic agents for optic nerve injury be efficiently screened, but also the therapeutic effect using the therapeutic agent may be maximized.

The foregoing description of the present disclosure is for illustrative purposes only, and one that has ordinary skill in the art to which the present disclosure belongs will understand that the present disclosure may be readily adapted to other specific forms without altering the technical ideas or essential features of the present disclosure. Therefore, the examples described above should be understood in all respects as illustrative and not restrictive.

## Claims

1. A composition for diagnosing an optic nerve injury disease, comprising a preparation for measuring an expression level of Syntaxin 12 (STX 12) or Delphilin protein, a fragment thereof, or a polynucleotide encoding the protein.

2. The composition of claim 1, wherein the optic nerve injury disease is traumatic optic neuropathy, ischemic optic neuropathy, compressive optic neuropathy, toxic optic neuropathy, Leber hereditary optic neuropathy, optic nerve transection, optic neuritis, neuromyelitis optica, or amblyopia.

3. A kit for diagnosing an optic nerve injury disease, comprising the composition of claim 1.

4. The kit of claim 3, wherein the kit is a reverse transcription polymerase chain reaction (RT-PCR) kit, a DNA chip kit, an enzyme linked immunosorbent assay (ELISA) kit, a protein chip kit, a rapid kit, or a multiple reaction monitoring (MRM) kit.

5. An information provision method for diagnosis or treatment of an optic nerve injury disease, comprising:
measuring an expression level of STX 12 or Delphilin protein, a fragment thereof, or a polynucleotide encoding the protein, in a biological sample isolated from a subject; and
comparing the measured expression level of the protein, the fragment thereof, or the polynucleotide encoding the protein with that of a normal control sample.

6. The method of claim 5, further comprising determining that the subject has an optic nerve injury disease, when the expression level is decreased as compared with the expression level of the normal control.

7. The method of claim 5, wherein the sample is retinal tissue.

8. A method of screening for a therapeutic agent for an optic nerve injury disease, comprising:
measuring an expression level of STX 12 or Delphilin protein, a fragment thereof, or a polynucleotide encoding the protein, in a biological sample isolated from a subject administered with a candidate substance for treatment of an optic nerve injury disease; and
comparing the measured expression level of the protein, the fragment thereof, or the polynucleotide encoding the protein with that of a control sample not administered with the candidate substance.

9. The method of claim 8, wherein the measurement of the expression level of the polynucleotide is performed by reverse transcription polymerase chain reaction, competitive reverse transcription polymerase chain reaction, real-time reverse transcription polymerase chain reaction, RNase protection assay, Northern blotting, or DNA chip.

10. The method of claim 8, wherein the measurement of the expression level of the protein or the fragment thereof is performed by protein chip analysis, immunoassay, ligand binding assay, matrix desorption/ionization time of flight mass spectrometry (MALDI-TOF) analysis, surface enhanced laser desorption/ionization time of flight mass spectrometry (SELDI-TOF) analysis, radioimmunoassay, radioimmunodiffusion, ouchterlony immunodiffusion, rocket immunoelectrophoresis, tissue immunostaining, complement fixation assay, two-dimensional electrophoresis analysis, liquid chromatography-mass spectrometry (LC-MS), liquid chromatography-mass spectrometry/mass spectrometry (LC-MS), western blot, enzyme linked immunosorbent assay (ELISA), or a combination thereof.
